# EUROPÄISCHE PATENTSCHRIFT

(11) **EP 4 105 636 B1**
(45) Veröffentlichungstag und Bekanntmachung des Hinweises auf die Patenterteilung: **08.01.2025**
(21) Anmeldenummer: 22178003.4
(22) Anmeldetag: 09.06.2022
(51) Int. Cl.: G01N 15/06

(54) **VORRICHTUNG UND VERFAHREN ZUR ERFASSUNG DER UNGEFÄHREN ANZAHL VON LEUKOZYTEN IN EINEM DIALYSAT NACH DESSEN VERWENDUNG IN DER PERITONEALDIALYSE**
DEVICE AND METHOD FOR DETECTING THE APPROXIMATE NUMBER OF LEUKOCYTES IN A DIALYSATE AFTER USE IN PERITONEAL DIALYSIS
APPAREIL ET PROCÉDÉ DE DÉTECTION DU NOMBRE APPROXIMATIF DE LEUCOCYTES DANS UNE DIALYSE APRÈS SON UTILISATION EN DIALYSE PÉRITONÉALE

(30) Priorität: 17.06.2021 DE 102021115737
(43) Veröffentlichungstag der Anmeldung: 21.12.2022
(73) Patentinhaber: Hinkel, Ulrich Paul, 99425 Weimar (DE); Becker, Franz Ferdinand, 63110 Rodgau (DE)
(72) Erfinder: Hinkel, Ulrich Paul, 99425 Weimar (DE); Becker, Franz Ferdinand, 63110 Rodgau (DE)
(74) Vertreter: Flosdorff, Jürgen

(56) Entgegenhaltungen:
- JP-A- H09 239 023
- US-A1- 2020 405 243

## Beschreibung

Die Erfindung betrifft eine Vorrichtung und ein Verfahren zur Erfassung der ungefähren Anzahl von Leukozyten und eines eventuellen Anstiegs der Leukozytenzahl in einem Dialysat nach dessen Verwendung in einer Peritonealdialyse und zur Analyse der gemessenen Werte.

Als Dialyse bezeichnet man ein medizinisches Verfahren, mit dem schädliche Stoffe aus dem Blut entfernt werden. Eingesetzt wird die Dialyse vor allem bei einer akuten oder chronischen Störung der Nierenfunktion. Bei der vorliegenden Erfindung geht es um die Bauchfelldialyse bzw. Peritonealdialyse. Bei dieser Dialyse findet der Blutreinigungsprozess über das Bauchfell statt, indem über einen Katheter eine Reinigungslösung (Dialysat) in die Bauchhöhle eingelassen und nach einer Verweildauer von ca. vier bis fünf Stunden oder länger wieder ausgelassen wird. In dieser Zeit gehen Schadstoffe aus dem Körper über das Bauchfell in das Dialysat über.

Die gefürchtetste Komplikation bei diesem Verfahren ist die Einschwemmung von Bakterien in die Bauchhöhle. Dies führt zu einer Bauchfellentzündung (Peritonitis). Diese Bauchfellentzündung zeigt sich durch eine Erhöhung von Zellen (Leukozyten) in der ausgelassenen Spüllösung. Normalerweise sind in der Spüllösung nur ein bis siebzig Leukozyten/µl vorhanden, wobei dies individuell unterschiedlich ist. Steigt die Anzahl der anfänglichen Leukozyten um über 100 Leukozyten/µl an, kann vom Beginn einer Peritonitis ausgegangen werden.

Mit 100 bis ca. 500 Leukozyten/µl ist der Beutel mit der ausgelassenen Spüllösung visuell noch klar. Erst bei ca. 1000 Leukozyten/µl kommt es zu einer für den Patienten sichtbaren Trübung, was dann einer fortgeschrittenen Bauchfellentzündung entspricht und viel zu spät ist. Bis heute muss der Patient, der die Peritonealdialyse bei sich zu Hause durchführt, die Trübung des Dialysats selbst erkennen, mit der Folge, dass das zu spät ist und es so fast immer zur stationären Aufnahme in eine Fachklinik kommt. In zu spät entdeckten Fällen liegt die Anzahl der Leukozyten häufig bei 20.000 bis 30.000 Leukozyten/µl. Eine Konzentration von 1.000 bis 2.000 Leukozyten/µl kann aber bereits zur irreversiblen Schädigung des Bauchfells führen.

Die DE 10 2017 008 012 A1 offenbart eine Vorrichtung zur Erfassung von Trübungen in einer wässrigen Spüllösung mit den Merkmalen des Oberbegriffs des Patentanspruchs 1. Bei der bekannten Vorrichtung ist eine Laserlichtquelle so angeordnet, dass ihr Licht durch den Schlauch verläuft und auf den gegenüberliegenden Detektor gerichtet ist, der eine Trübung der durch den Schlauch fließenden Flüssigkeit erfassen kann. Die erfasste Lichtmenge gibt Aufschluss über eine Trübung oder Luminiszenz der Flüssigkeit und wird von einer Auswerteeinrichtung analysiert, die die so ermittelten Messwerte anzeigt. Durch Zugabe eines entsprechenden Indikators zu der Flüssigkeit können beispielsweise Leukozyten identifziert werden.

Die US 8,777,891 B2 offenbart eine Vorrichtung und ein Verfahren zur Erfassung eines frühen Zustands einer beginnenden Peritonitis, bei der eine Lichtdetektoreinrichtung nur die von streuenden Oberflächen von Leukozyten reflektierte Streustrahlung erfasst, indem eine Laserlichtquelle parallel zur Lichtdetektoreinrichtung angeordnet ist, und den Strahl der Laserlichtquelle in Längsrichtung durch den Schlauch richtet.

Die US2020/405243 A1 offenbart ein System zur Überwachung einer Flüssigkeit. In einer Ausführungsform sind zwei Lichtquellen im Winkel von 90° zueinander angeordnet, die ihr Licht auf eine zu untersuchende Flüssigkeit in einem Schlauch richten. Den Lichtquellen gegenüberliegend sind zwei optische Sensoren angeordnet, die die Lichtintensität der beiden Lichtquellen getrennt voneinander im Winkel von 180° und 90° messen. Diese Messwerte werden in zwei mathematische Gleichungen eingesetzt und ergeben so einen Schätzwert für die Trübung der Flüssigkeit. In einer anderen Ausführungsform sind zwei Lichtquellen einander gegenüberliegend angeordnet und zwei optische Sensoren befinden sich einander gegenüberliegend um 90° versetzt zu den Lichtquellen. Diese Sensoren messen die 90°-Streustrahlung der beiden Lichtquellen. Auf diese Weise soll die Trübung in der durchfließenden Flüssigkeit hochgradig genau ermittelt werden.

Der vorliegenden Erfindung liegt die Aufgabe zugrunde, eine Vorrichtung und ein Verfahren zur Erfassung der ungefähren Anzahl von Leukozyten in einem Dialysat nach dessen Verwendung bei der Peritonealdialyse anzugeben, mit der bzw. dem ein Anstieg um etwa zehn Leukozyten/µl erkennbar ist und individuell einer beginnenden Peritonitis zugeordnet werden kann, so dass einem schweren Verlauf bereits in der Entwicklungsphase vorzukommen ist, und frühzeitig ein Antibiotikum eingesetzt werden kann.

Diese Aufgabe wird erfindungsgemäß durch die Merkmale der Patentansprüche 1 und 9 gelöst.

Vorteilhafte Ausgestaltungen der Erfindung sind in den Unteransprüchen gekennzeichnet.

Die erfindungsgemäße Vorrichtung enthält ein Gehäuse mit einer Durchgangsöffnung zum Einlegen eines lichtdurchlässigen Schlauchs, durch den das zu untersuchende Dialysat strömt, wobei der Schlauch mit einer Schlauchklemmeinrichtung in dem Gehäuse fixierbar ist, wenigstens eine auf den Schlauch gerichtete Laserlichtquelle, eine Lichtdetektoreinrichtung mit einer Signal-Auswerteeinrichtung, ein Display zur Anzeige der ermittelten Werte und eine Stromversorgung für die Lichtquelle, die Lichtdetektoreinrichtung und das Display, wobei die Laserlichtquelle und die Lichtdetektoreinrichtung so im Winkel zueinander angeordnet sind, dass die Lichtdetektoreinrichtung nur die von den streuenden Oberflächen fester Partikel reflektierte Streustrahlung erfasst. Dabei befinden sich in dem ausgelassenen Dialysat hauptsächlich oder ausschließlich Leukozyten, so dass die Streustrahlung zur Identifikation der ungefähren Anzahl von Leukozyten geeignet ist. Es wurde festgestellt, dass die Messreihen im unteren Konzentrationsbereich von 0 bis 500 Leukozyten/µl liegen und dabei entsprechenden Laboranalysen sehr nahe kommen.

Wenn ein Patient die erfindungsgemäße Vorrichtung erhält, werden zunächst die Ausgangswerte der Streustrahlung erfasst. Wenn der Anfangswert bei ca. 5 Leukozyten/µl liegt und im Laufe von weiteren Tests im Rahmen von etwa zwei Wochen ein Anstieg auf maximal 20 Leukozyten/µl erfasst wird, ist ein weiterer Anstieg auf 50 Leukozyten noch als ungefährlich einzustufen. Ein plötzlicher Anstieg auf 100 Leukozyten/µl wäre hingegen ein Alarmsignal für eine voranschreitende Entzündung, so dass diagnostische und therapeutische Maßnahmen ergriffen werden müssen. Wenn bei einem anderen Patienten die Anfangswerte bei 50 bis 70 Leukozyten/µl liegen, ist ein Anstieg auf 100 Leukozyten/µl kein Zeichen für eine Entzündung, sondern dies ist erst bei einem deutlich höheren Wert der Fall. Der kritische Anstieg der Anzahl von erfassten Leukozyten muss individuell für jeden Nutzer der Vorrichtung festgelegt werden.

Mit Vorteil ist vorgesehen, dass die Lichtdetektoreinrichtung ein Kameramodul mit integrierter Signalverarbeitung und mit einem Kameraobjektiv ist. Bei dem Kameramodul kann es sich um einen Farbkamerasensor handeln.

Die Laserlichtquelle ist vorteilhafterweise steuerbar und kann mit einer Strahlformungsoptik versehen sein.

Die wenigstens eine Laserlichtquelle kann eine rote Laserdiode sein.

Außerdem kann eine LED-Lichtquelle angeordnet sein, die ihr Licht direkt auf die Lichtdetektoreinrichtung richtet, so dass diese die Transmissionslichtstärke des Dialysats misst. Wie bereits oben erwähnt ist, ist das abfließende Dialysat visuell klar, wenn sich weniger als ca. 1.000 Leukozyten/µl in dem Dialysat befinden, wobei es erst danach zu einer sichtbaren Trübung kommt. Diese Trübung kann aber dazu führen, dass der Helligkeitsgrad der Streustrahlung verringert wird, so dass der ermittelte Wert der Streustrahlung auf eine zu geringe Anzahl von Leukozyten schließen lässt. Je nach Grad der Trübung ist die ungefähre Anzahl von Leukozyten gegenüber dem gemessenen Streulicht zu erhöhen.

Mittels der Transmissionsmessung ist es im Gegensatz zur Streustrahlungsmessung möglich, die Beschaffenheit und das Spektrum der Auslaufflüssigkeit zu analysieren und zu bestimmen, ob die laufende Peritonealdialyse abgebrochen werden soll, wenn das Bauchfell von dem Dialysat angegriffen und abgenutzt wird, was anhand der Farbgebung des Dialysats abgeleitet werden kann. Um eine derartige Transmissionsmessung durchzuführen, kann das Licht der LED-Lichtquelle vorteilhafterweise abwechselnd rot, grün oder blau sein.

Zweckmäßigerweise sind alle Bauteile im Inneren des Gehäuses angeordnet.

Das erfindungsgemäße Verfahren sieht die Bereitstellung der oben beschriebenen Vorrichtung vor, wobei das durch den Schlauch fließende Dialysat vorzugsweise täglich in mehreren Intervallen beleuchtet wird, um einen evtl. Anstieg der Anzahl von Leukozyten zu erfassen. Die Messdauer kann jeweils ca. fünf Sekunden lang sein. Mit Vorteil wird vorgeschlagen, dass zu Beginn einer Messreihe der Helligkeitswert ohne interne Beleuchtung gemessen wird. Hierdurch lässt sich der Einfluss der Helligkeit der Umgebung des Gehäuses erkennen und eliminieren.

Vorteilhafterweise wird das nach einer Peritonealdialyse durch einen Schlauch abfließende Dialysat in der erfindungsgemäßen Vorrichtung beleuchtet. Das Dialysat kann aber auch in einem Beutel aufgefangen werden und erst dann, also abgekoppelt vom Patienten durch einen Schlauch abgeführt werden, der in das Gehäuse der Vorrichtung eingelegt wird.

Die erfindungsgemäße Vorrichtung kann mit einer telemedizinischen Anbindungsmöglichkeit ausgerüstet werden, durch die automatisch eine

Benachrichtigung an einen behandelnden Arzt übermittelt wird, wenn ein kritischer Anstieg der Leukozyten festgestellt wird.

Weitere Einzelheiten der Erfindung ergeben sich aus der nachfolgenden Beschreibung einer bevorzugten Ausführungsform sowie anhand der Zeichnungen. Dabei zeigen:
- Figur 1: das Messprinzip der Leukozytenbestimmung in einer schematischen Darstellung;
- Figur 2: eine Ausführungsform des erfindungsgemäßen Messgeräts in auseinandergezogener Darstellung.

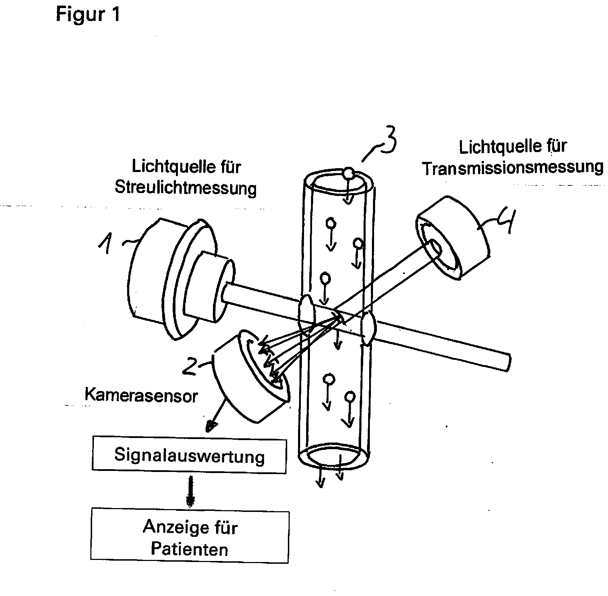

In Figur 1 ist eine Lichtlaserquelle 1 in einem Winkel von etwa 30° bis 90° zur Mittelachse eines Kamerasensors 2 seitlich neben einem durchsichtigen Schlauch 3 angeordnet, durch den das zu untersuchende Dialysat fließt. Eine LED-Lichtquelle 4 ist für eine Transmissionslichtmessung direkt auf den Kamerasensor 2 gerichtet. Der Kamerasensor 3 steht mit einer Signalauswertung 5 und einer Anzeigeeinrichtung 6 für Patienten in Verbindung.

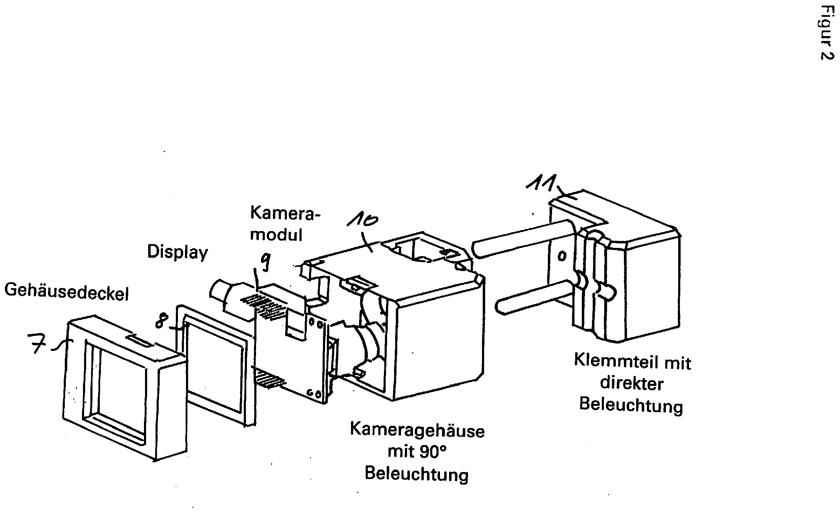

Die Ausführungsform des Messgeräts gemäß Figur 2 enthält einen Gehäusedeckel 7, ein Display 8, ein Kameramodul 9, ein Kameragehäuse 10 mit 90° Beleuchtung und ein Klemmteil 11 mit der direkten Beleuchtung.

Es wird betont, dass die Erfindung nicht auf die beschriebenen und dargestellten Ausführungsformen beschränkt ist. Vielmehr sind alle offenbarten Merkmale auf jede sinnvolle Weise einzeln miteinander kombinierbar.

## Patentansprüche

1. Vorrichtung zur Erfassung der ungefähren Anzahl von Leukozyten in einem Dialysat nach deren Verwendung in einer Peritonealdialyse, und zu deren Analyse,
mit einem Gehäuse mit einer Durchgangsöffnung zum Einlegen eines lichtdurchlässigen Schlauchs (3) und mit einer integrierten Schlauchklemmeinrichtung,
wenigstens einer auf den Schlauch (3) gerichtete Laserlichtquelle (1), einer Lichtdetektoreinrichtung (9) mit einer Signal-Auswerteeinrichtung, einem Display (8) und
mit einer Stromversorgung für die Lichtquelle, die Lichtdetektoreinrichtung mit der Lichtsignal-Auswerteeinrichtung und das Display,
wobei die Laserlichtquelle (1) und die Lichtdetektoreinrichtung (2) so im Winkel zueinander angeordnet sind, dass die Lichtdetektoreinrichtung (2) nur die von streuenden Oberflächen fester Partikel reflektierte Streustrahlung erfasst, **dadurch gekennzeichnet,**
**dass** außerdem eine LED-Lichtquelle (4) angeordnet ist, die ihr Licht auf die Lichtdetektoreinrichtung (2) richtet, die die Transmissionslichtstärke misst und nach Erfassung einer Trübung des Dialysats den Wert der erfassten Streustrahlung entsprechend erhöht.

2. Vorrichtung nach Anspruch 1,
**dadurch gekennzeichnet,**
**dass** die Lichtdetektoreinrichtung ein Kameramodul (9) mit integrierter Signalverarbeitung und mit einem Kameraobjektiv ist.

3. Vorrichtung nach Anspruch 2,
**dadurch gekennzeichnet,**
**dass** das Kameramodul ein Farbkamerasensor ist.

4. Vorrichtung nach einem der Ansprüche 1 bis 3,
**dadurch gekennzeichnet,**
**dass** die wenigstens eine Laserlichtquelle (1) steuerbar mit Strahlformungsoptik ist.

5. Vorrichtung nach einem der Ansprüche 1 bis 4,
**dadurch gekennzeichnet,**
**dass** die wenigstens eine Laserlichtquelle eine rote Laserdiode ist.

6. Vorrichtung nach Anspruch 1,
**dadurch gekennzeichnet,**
**dass** das Licht der LED-Lichtquelle grün ist.

7. Vorrichtung nach Anspruch 1,
**dadurch gekennzeichnet,**
**dass** das Licht der LED-Lichtquelle abwechselnd rot, grün oder blau ist.

8. Vorrichtung nach einem der Ansprüche 1 bis 7,
**dadurch gekennzeichnet,**
**dass** alle Bauteile in dem Gehäuse angeordnet sind.

9. Verfahren zur Erfassung der ungefähren Anzahl von Leukozyten in einem Dialysat nach dessen Verwendung in einer Peritonealdialyse, und zu dessen Analyse,
**dadurch gekennzeichnet,**
**dass** eine Vorrichtung nach einem der Ansprüche 1 bis 8 bereitgestellt wird und
**dass** das durch den Schlauch fließende Dialysat täglich in mehreren Intervallen beleuchtet wird.

10. Verfahren nach Anspruch 9,
**dadurch gekennzeichnet,**
**dass** die Messdauer jeweils ca. 5 sec lang ist.

11. Verfahren nach einem der Ansprüche 9 oder 10,
**dadurch gekennzeichnet,**
**dass** zu Beginn einer Messreihe der Helligkeitswert ohne interne Beleuchtung gemessen wird.

12. Verfahren nach einem der Ansprüche 9 bis 11.
**dadurch gekennzeichnet,**
**dass** das nach einer Peritonealdialyse abfließende Dialysat beleuchtet wird.

## Claims

1. Apparatus for determining the approximate number of leukocytes in a dialysate after its use in peritoneal dialysis and for its analysis, including a housing with an opening for the insertion of a translucent tube (3) and including an integrated tube clamping device, at least one laser light source (1) directed onto the tube (3), a light detector device (9) with a signal analysis device, a display (8) and including a power supply for the light source, the light detector device with the light signal analysis device and the display, wherein the laser light source (1) and the light detector device (2) are so arranged at an angle to one another that the light detector device (2) only detects the scattered radiation reflected by scattering surfaces of solid particles, **characterised in that** an LED light source (4) is further provided, which directs its light onto the light detector device (2), which measures the transmitted light intensity and, after detecting clouding of the dialysate, correspondingly increases the value of the detected scattered radiation.

2. Apparatus as claimed in Claim 1, **characterised in that** the light detector device is a camera module (9) with integrated signal processing and with a camera objective.

3. Apparatus as claimed in Claim 2, **characterised in that** the camera module is a colour camera sensor.

4. Apparatus as claimed in one of Claims 1 to 3, **characterised in that** the at least one laser light source (1) is controllable with beam formation optics.

5. Apparatus as claimed in one of Claims 1 to 4, **characterised in that** the at least one laser light source is a red laser diode.

6. Apparatus as claimed in Claim 1, **characterised in that** the light from the LED light source is green.

7. Apparatus as claimed in Claim 1, **characterised in that** the light from the LED light source is alternating red, green or blue.

8. Apparatus as claimed in one of Claims 1 to 7, **characterised in that** all the components are arranged in a housing.

9. A method of detecting the approximate number of leukocytes in a dialysate after its use in peritoneal dialysis and for analysing it, **characterised in that** an apparatus as claimed in one of Claims 1 to 8 is provided and that the dialysate flowing through the tube is illuminated daily in a number of intervals.

10. A method as claimed in Claim 8, **characterised in that** the measurement duration is in each case ca. 5 sec long.

11. A method as claimed in one of Claims 9 or 10, **characterised in that** at the beginning of a measurement series the brightness value is measured without internal illumination.

12. A method as claimed in one of Claims 9 to 11, **characterised in that** the dialysate flowing away after peritoneal dialysis is illuminated.

## Revendications

1. Dispositif permettant de détecter et d'analyser le nombre approximatif de leucocytes dans un dialysat après son utilisation dans une dialyse péritonéale,
avec un boîtier doté d'une ouverture de passage pour l'insertion d'un tuyau transparent (3) et d'un dispositif de serrage de tuyau intégré,
au moins une source de lumière laser (1) dirigée sur le tuyau (3), un dispositif détecteur de lumière (9) avec un dispositif d'évaluation de signal, un écran (8) et
avec une alimentation électrique pour la source de lumière, le dispositif détecteur de lumière avec le dispositif d'évaluation du signal lumineux et l'écran, dans lequel
la source de lumière laser (1) et le dispositif détecteur de lumière (2) sont disposés selon un angle l'un par rapport à l'autre de sorte que le dispositif détecteur de lumière (2) ne détecte que le rayonnement diffusé réfléchi par des surfaces dispersantes de particules solides, **caractérisé en ce**
**qu'**une source de lumière LED (4) est en outre disposée, laquelle dirige sa lumière sur le dispositif détecteur de lumière (2) qui mesure l'intensité de la lumière transmise et augmente en conséquence la valeur du rayonnement diffus détecté après détection d'une turbidité du dialysat.

2. Dispositif selon la revendication 1, **caractérisé en ce**
**que** le dispositif détecteur de lumière est un module de caméra (9) avec traitement de signal intégré et avec un objectif de caméra.

3. Dispositif selon la revendication 2, **caractérisé en ce**
**que** le module de caméra est un capteur de caméra couleur.

4. Dispositif selon l'une quelconque des revendications 1 à 3,
**caractérisé en ce**
**que** l'au moins une source de lumière laser (1) peut être commandée avec une optique de formation de faisceau.

5. Dispositif selon l'une quelconque des revendications 1 à 4,
**caractérisé en ce**
**que** l'au moins une source de lumière laser est une diode laser rouge.

6. Dispositif selon la revendication 1, **caractérisé en ce**
**que** la lumière de la source de lumière LED est verte.

7. Dispositif selon la revendication 1, **caractérisé en ce**
**que** la lumière de la source de lumière LED est alternativement rouge, verte ou bleue.

8. Dispositif selon l'une quelconque des revendications 1 à 7,
**caractérisé en ce**
**que** tous les composants sont disposés dans le boîtier.

9. Procédé permettant de détecter et d'analyser le nombre approximatif de leucocytes dans un dialysat après utilisation dans une dialyse péritonéale,
**caractérisé en ce**
**qu'**un dispositif selon l'une quelconque des revendications 1 à 8 est fourni et
**que** le dialysat s'écoulant à travers le tube est éclairé quotidiennement à plusieurs intervalles.

10. Procédé selon la revendication 9,
**caractérisé en ce**
**que** la durée de mesure est respectivement d'environ 5 secondes.

11. Procédé selon l'une quelconque des revendications 9 ou 10,
caractérisé en ce
au début d'une série de mesures, la valeur de luminosité est mesurée sans éclairage interne.

12. Procédé selon l'une quelconque des revendications 9 à 11.
**caractérisé en ce**
**que** le dialysat s'écoulant après une dialyse péritonéale est éclairé.
